(19) Europäisches Patentamt
European Patent Office
Office européen des brevets

(11) Veröffentlichungsnummer: **0 342 482 B1**

(12) **EUROPÄISCHE PATENTSCHRIFT**

(45) Veröffentlichungstag der Patentschrift: **03.08.94**

(51) Int. Cl.$^5$: **C07D 415/00**

(21) Anmeldenummer: **89108309.9**

(22) Anmeldetag: **09.05.89**

# (54) Verfahren zur Herstellung von Pyrimidinderivaten.

(30) Priorität: **17.05.88 CH 1857/88**

(43) Veröffentlichungstag der Anmeldung:
**23.11.89 Patentblatt 89/47**

(45) Bekanntmachung des Hinweises auf die Patenterteilung:
**03.08.94 Patentblatt 94/31**

(84) Benannte Vertragsstaaten:
**AT BE CH DE FR GB IT LI NL**

(56) Entgegenhaltungen:
**DE-A- 2 040 554**

**CHEMICAL ABSTRACTS, Band 67, Nr. 5, 31. Juli 1967, Seite 2091, ZusammenfassungNr. 21885r, Columbus, Ohio, US; Y. OKA et al.: "Studies on vitamin B1 and related compounds. CVI. A novel synthesis of hydroxyethylthiamine"**

(73) Patentinhaber: **F. HOFFMANN-LA ROCHE AG**
**Postfach 3255**
**CH-4002 Basel(CH)**

(72) Erfinder: **Hengartner, Urs, Dr.**
**Binzenstrasse 28**
**CH-4058 Basel(CH)**
Erfinder: **Moine, Gérard, Dr.**
**180 Lafayette Avenue**
**Apt.10E**
**Passaic, N.J. 07055(US)**

(74) Vertreter: **Cottong, Norbert A. et al**
**Grenzacherstrasse 124**
**Postfach 3255**
**CH-4002 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 342 482 B1

## Beschreibung

Die vorliegende Erfindung betrifft ein neues Verfahren zur Herstellung eines Pyrimidinderivates, bzw. von Thiamin (Vitamin B1), welches Verfahren vom bekannten Grewe-Diamin (2-Methyl-4-amino-5-aminomethyl-pyrimidin) ausgeht.

Das erfindungsgemässe Verfahren zur Herstellung eines Pyrimidinderivats bzw. von Thiamin aus dem Grewe-Diamin der Formel

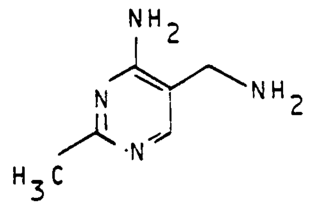

I

ist dadurch gekennzeichnet, dass man das Grewe-Diamin mit einem Orthoameisensäureester der allgemeinen Formel

$HC(OR)_3$ II

worin R einen Alkylrest mit 1-4 C-Atomen darstellt, in Gegenwart eines sauren Katalysators erhitzt, dass man, gewünschtenfalls, das so erhaltene Pyrimidinderivat der Formel

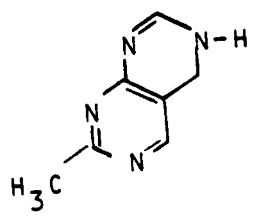

III

mit einem Keton der allgemeinen Formel

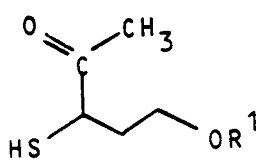

IV

worin $R^1$ Wasserstoff oder einen Acylrest mit 1-4 C-Atomen darstellt,
in Gegenwart einer aliphatischen Carbonsäure mit 1-4 C-Atomen umsetzt, und die erhaltene Verbindung der allgemeinen Formel

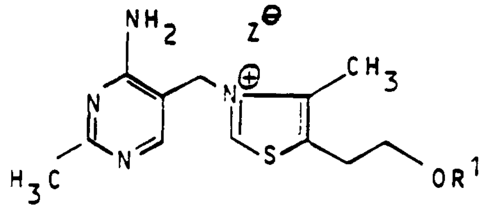

V

worin $R^1$ die obige Bedeutung hat und $Z^-$ das Anion der verwendeten aliphatischen Carbonsäure darstellt,
durch Behandlung mit einer anorganischen Säure in das Thiamin der Formel

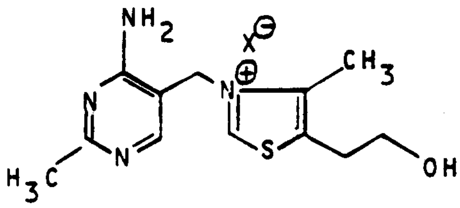

VI

worin $X^-$ $Cl^-$, $Br^-$ oder $NO_3^-$ darstellt, oder ein Salz hiervon überführt.

Der Ausdruck "Alkylrest mit 1-4 C-Atomen" bedeutet im Rahmen der vorliegenden Erfindung sowohl geradkettige als auch verzweigte Alkylreste wie Methyl, Aethyl, n-Propyl, Isopropyl, n-Butyl, Isobutyl und tert.Butyl. Der Ausdruck "Acylrest mit 1-4 C-Atomen" bedeutet Reste von geradkettigen oder verzweigten Alkancarbonsäuren mit 1-4 C-Atomen, wie Ameisensäure, Essigsäure, Propionsäure, n-Buttersäure und Isobuttersäure.

Die Umsetzung des Grewe-Diamins mit einem Orthoameisensäureester der Formel II erfolgt erfindungsgemäss durch Erhitzen in Gegenwart eines sauren Katalysators. Dies erfolgt zweckmässig bei einer Temperatur von etwa 70 °C bis etwa 200 °C, insbesondere von etwa 90 °C bis etwa 150 °C und vorzugsweise von etwa 100 °C bis etwa 120 °C. Sofern der verwendete Orthoameisensäureester einen zu niedrigen Siedepunkt aufweist, muss die Reaktion in einem geschlossenen Gefäss unter Druck durchgeführt werden. Als saure Katalysatoren kommen im Rahmen der vorliegenden Erfindung sowohl anorganische als auch organische Säuren in Betracht. Als Beispiele derartiger anorganischer Säuren können insbesondere genannt werden Schwefelsäure, Halogenwasserstoffsäuren wie z.B. Chlorwasserstoffsäure und Bromwasserstoffsäure, oder auch Phosphorsäure und dergleichen. Als organische Säuren können insbesondere genannt werden Carbonsäuren mit 1 bis 4 C-Atomen, wie etwa Ameisensäure, Essigsäure, Propionsäure und dergleichen, sowie auch Sulfonsäuren wie etwa Benzolsulfonsäure und insbesondere p-Toluolsulfonsäure und dergleichen.

Von den verwendeten Orthoameisensäureestern sind der Methylester und der Aethylester bevorzugt, insbesondere aber der Aethylester.

Die Umsetzung des Pyrimidinderivats der Formel III mit einem Keton der allgemeinen Formel IV erfolgt erfindungsgemäss in Gegenwart einer aliphatischen Carbonsäure mit 1-4 C-Atomen. Als solche kommen sowohl geradkettige wie auch verzweigte Carbonsäure in Frage, wie Ameisensäure, Essigsäure, Propionsäure, n-Buttersäure und Isobuttersäure. Bevorzugte Carbonsäure sind in diesem Zusammenhang Ameisensäure und Essigsäure, insbesondere Ameisensäure. Der Druck und die Temperatur sind bei dieser Umsetzung keine kritischen Grössen. So kann diese ohne weiteres bei Normaldruck durchgeführt werden. Die Temperatur liegt zweckmässig zwischen etwa 0 °C und etwa 80 °C, vorzugsweise zwischen etwa 20 °C und etwa 50 °C. Bevorzugt wird diese Reaktion unter Normaldruck und bei Raumtemperatur durchgeführt.

Die Ueberführung einer Verbindung der allgemeinen Formel V in das Thiamin der Formel VI erfolgt durch Behandlung mit einer anorganischen Säure, wobei die in der Verbindung der Formel V vorhandene Estergruppe zur Hydroxylgruppe verseift wird. Als anorganische Säuren kommen hier in Frage Chlorwasserstoffsäure, Bromwassestoffsäure und Salpetersäure. Diese Behandlung kann in an sich bekannter Weise erfolgen, zweckmässig in alkoholischer Lösung, vorzugsweise in methanolischer oder äthanolischer Lösung.

Die nachfolgenden Beispiele erläutern die Erfindung.

Beispiel 1

In einem 500 ml Zweihalskolben, versehen mit einem Thermometer und einer 20 cm Vigreux-Kolonne mit Liebigkühler, werden 69 g (0,5 Mol) Grewe-Diamin, 148,2 g (1 Mol) Triäthylorthoformat und 2,5 g p-Toluolsulfonsäure gegeben. Die gerührte Suspension wird langsam auf ca. 110 °C erhitzt, sodass die Temperatur am Kopf der Vigreux-Kolonne 80-85 °C erreicht. Aethanol wird dann abdestilliert bis das Reaktionsgemisch in dem Kolben eine feste Masse wird. Die Temperatur wird während 30 Minuten bei 100-110 °C gehalten, dann werden 250 ml Toluol zugegeben und die erhaltene Suspension eine weitere Stunde bei 90 °C gerührt. Das Reaktionsgemisch wird dann auf Raumtemperatur abgekühlt und über Nacht in den Kühlschrank gestellt. Der hellbraune Niederschlag wird dann abfiltriert und zweimal mit je 50 ml Toluol gewaschen. Das erhaltene Rohmaterial wird bei 50 °C unter reduziertem Druck (Wasserstrahlvakuum) getrocknet und man erhält 59,3g eines beige-farbenen Feststoffes, welcher sodann bei 150 °C (Oelbad-Temperatur) im Hochvakuum (0,3 mbar) sublimiert wird. Man erhält so 52,5 g (0,35 Mol; 71%) 5,6-Dihydro-2-methylpyrimido[4,5-d]pyrimidin als weissen Feststoff mit einem Schmelzpunkt von 182 °C (Zers.).

Beispiel 2

In einem 250 ml Dreihalskolben, versehen mit Thermometer, Magnetrührer und 50 ml Tropftrichter, werden 100 ml Ameisensäure sowie 7,4 g (0,05 Mol) 5,6-Dihydro-2-methylpyrimido[4,5-d]pyrimidin gegeben. Zu der erhaltenen gelblichen Lösung werden sofort 9,25 g (0,052 Mol) 3-Mecapto-4-oxopentylacetat zugegeben, dass die Temperatur nie 35-40 °C übersteigt. Das Reaktionsgemisch wird weitere 30 Minuten bei Raumtemperatur gerührt, worauf 25 ml einer frisch zubereiteten gesättigten Lösung von HCl in absolutem Aethanol tropfenweise zugesetzt werden. Die Temperatur steigt hierbei auf 35-36 °C. Anschliessend wird das Reaktionsgemisch noch weitere 30 Minuten bei Raumtemperatur gerührt. Das rohe Reaktionsgemisch wird dann in einen 500 ml Kolben gegeben und bei 50 °C unter vermindertem Druck (Wasserstrahlvakuum) eingedampft und man erhält 26,07 g eines grün-gelblichen Rückstandes, welcher in 100 ml absolutem Aethanol aufgenommen wird. Hierauf werden 30 ml einer 25%igen wässrigen HCl-Lösung zugegeben und das Reaktionsgemisch auf einem Dampfbad erhitzt bis eine klare Lösung vorliegt. Diese Lösung wird auf Raumtemperatur abgekühlt und über Nacht in den Kühlschrank gestellt. Die erhaltenen weissen Kristalle werden abgetrennt und im Wasserstrahlvakuum getrocknet. Man erhält 14,56 g (86,3%) Thiamin-hydrochlorid mit einem Schmelzpunkt von 245-246 °C (Zers.). Die Mutterlauge wird dann bei 50 °C unter vermindertem Druck (Wasserstrahlvakuum) eingedampft und der Rückstand in 50 ml Wasser aufgenommen. Die wässrige Phase wird dann zweimal mit je 25 ml Dichlormethan gewaschen und im Wasserstrahlvakuum eingedampft. Man erhält 3,39 g eines noch leicht grünlichen Rückstandes, welcher wiederum in 20 ml absolutem Aethanol aufgenommen wird. 5 ml einer 25%igen wässrigen HCl-Lösung werden zugesetzt und das Reaktionsgemisch auf einem Dampfbad erhitzt bis eine klare Lösung entsteht. Diese wird auf Raumtemperatur abgekühlt und über Nacht im Kühlschrank aufbewahrt. Die erhaltenen weissen Kristalle werden abfiltriert und man erhält weitere 1,42 g (8,4%) Thiamin-hydrochlorid mit einem Schmelzpunkt von 244-245 °C (Zers.).

Das als Ausgangsmaterial verwendete 3-Mercapto-4-oxopentylacetat kann wie folgt hergestellt werden:

In einen 250 ml Dreihalskolben, versehen mit Thermometer, Magnetrührer und 100 ml Tropftrichter, werden 50 ml absolutes Methanol und 7,22 g (0,1 Mol) wasserfreies Kaliumhydrogensulfid gegeben. Der Kolben wird in einem Eisbad auf 0 °C abgekühlt und 17,9 g (0,1 Mol) 3-Chlor-4-oxopentylacetat, gelöst in 50 ml absolutem Methanol, werden tropfenweise zugesetzt, wobei die Temperatur in dem Kolben zwischen 0 und 5 °C gehalten wird. Nach vollständiger Zugabe, wird das Rühren noch eine Stunde bei Raumtemperatur fortgesetzt und Stickstoff wird durch das Gemisch geleitet um rückständigen Schwefelwasserstoff zu entfernen. Das ausgefallenen KCl wird abfiltriert und das Lösungsmittel unter Wasserstrahlvakuum abdestilliert. Der Rückstand wird in 50 ml Dichlormethan aufgenommen und unlösliches Material abfiltriert. Nach Abdampfen des Lösungsmittels im Wasserstrahlvakuum bei 30 °C erhält man 14,9 g einer leicht gelblichen Flüssigkeit. Durch Kugelrohrdestillation des rohen Reaktionsgemisches bei 120 °C/0,4 mbar erhält man 12,95 g (0,07 Mol; 73,5%) 3-Mercapto-4-oxopentylacetat als farblose Flüssigkeit.

Beispiel 3

In einem 250 ml Dreihalskolben, versehen mit Thermometer, Magnetrührer und 50 ml Tropftrichter, werden 100 ml Ameisensäure sowie 7,4g (0,05 Mol) 5,6-Dihydro-2-methylpyrimido[4,5-d]pyrimidin gegeben. Zu der erhaltenen gelblichen Lösung werden sofort 9,25 g (0,052 Mol) 3-Mercapto-4-oxopentylacetat so zugegeben, dass die Temperatur nie 35-40 °C übersteigt. Das Reaktionsgemisch wird weitere 30 Minuten bei Raumtemperatur gerührt und dann in einen 500 ml Kolben gegossen. Die Ameisensäure wird bei 50 °C am Wasserstrahlvakuum abgedampft. Der zurückbleibende, gelbe ölige Rückstand wird in 50 ml absolutem Methanol aufgenommen, dann werden 200 ml Diäthyläther zugesetzt und der Kolben für eine Stunde in den Kühlschrank gestellt. Der weisse Niederschlag wird abfiltriert, mit 50 ml absolutem Diäthyläther gewaschen und dann bei Raumtemperatur im Wasserstrahlvakuum getrocknet. Man erhält 19,84 g (0,045 Mol; 89,3%) O-Acetyl-thiamin-formiat (•2HCOOH) als weissen Feststoff.

**Patentansprüche**

**1.** Verfahren zur Herstellung eines Pyrimidinderivates, bzw. von Thiamin aus dem Grewe-Diamin der Formel

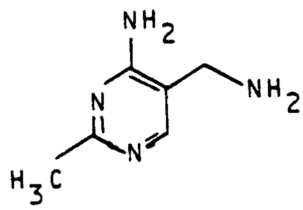

I

dadurch gekennzeichnet, dass man das Grewe-Diamin mit einem Orthoameisensäureester der allgemeinen Formel

$HC(OR)_3$ II

worin R einen Alkylrest mit 1-4 C-Atomen darstellt, in Gegenwart eines sauren Katalysators erhitzt, dass man, gewünschtenfalls, das so erhaltene Pyrimidinderivat der Formel

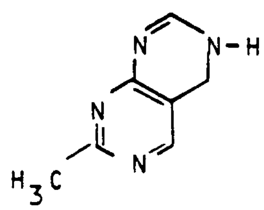

III

mit einem Keton der allgemeinen Formel

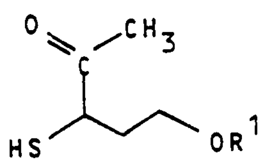

IV

worin $R^1$ Wasserstoff oder einen Acylrest mit 1-4 C-Atomen darstellt, in Gegenwart einer aliphatischen Carbonsäure mit 1-4 C-Atomen umsetzt, und die erhaltene Verbindung der allgemeinen Formel

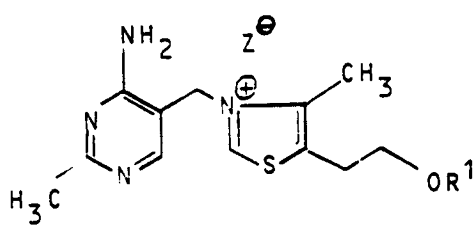

V

worin $R^1$ die obige Bedeutung hat und $Z^-$ das Anion der verwendeten aliphatischen Carbonsäure darstellt, durch Behandlung mit einer anorganischen Säure in das Thiamin der Formel

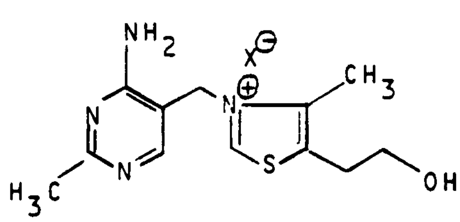

VI

worin $X^-$ $Cl^-$, $Br^-$ oder $NO_3^-$ darstellt, oder ein Salz hiervon überführt.

2. Verfahren gemäss Anspruch 1, dadurch gekennzeichnet, dass man die Umsetzung des Grewe-Diamins der Formel I mit einem Orthoameisensäureester der Formel II bei einer Temperatur von etwa 70 °C bis etwa 200 °C, insbesondere von etwa 90 °C bis etwa 150 °C und vorzugsweise von etwa 100 °C bis etwa 120 °C durchführt.

3. Verfahren gemäss Anspruch 1 oder 2, dadurch gekennzeichnet, dass man als sauren Katalysator Ameisensäure oder Essigsäure verwendet.

4. Verfahren gemäss Anspruch 3, dadurch gekennzeichnet, dass man als sauren Katalysator p-Toluolsulfonsäure verwendet.

5. Verfahren gemäss einem der Ansprüche 1 bis 4, dadurch gekennzeichnet, dass man als Orthoameisensäureester der Formel II den Methylester oder den Aethylester verwendet.

6. Verfahren gemäss einem der Ansprüche 1 bis 5, dadurch gekennzeichnet, dass man die Umsetzung des Pyrimidinderivates der Formel III mit einem Keton der Formel IV in Gegenwart von Ameisensäure durchführt.

## Claims

1. A process for the manufacture of a pyrimidine derivative and of thiamine from Grewe diamine of the formula

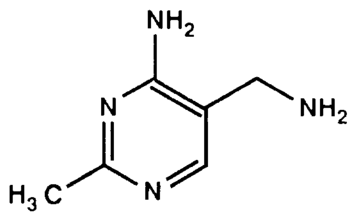

I

characterized by heating Grewe diamine with an orthoformic acid ester of the general formula

$HC(OR)_3$ II

wherein R represents an alkyl residue with 1-4 C atoms, in the presence of an acid catalyst, if desired reacting the thus-obtained pyrimidine derivative of the formula

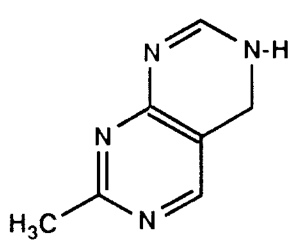

III

with a ketone of the general formula

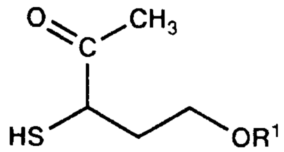

IV

wherein $R^1$ represents hydrogen or an acyl residue with 1-4 C atoms,
in the presence of an aliphatic carboxylic acid with 1-4 C atoms and converting the resulting compound of the general formula

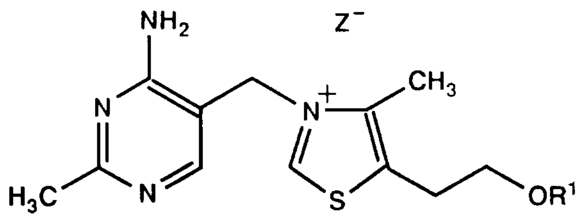

V

wherein $R^1$ has the above significance and $Z^-$ represents the anion of the aliphatic carboxylic acid which is used,
by treatment with an inorganic acid into thiamine of the formula

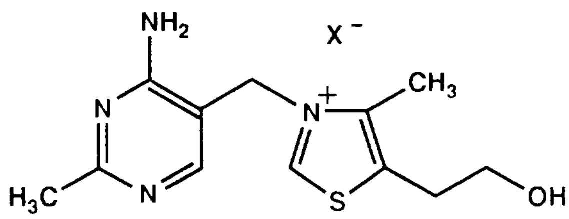

VI

wherein $X^-$ represents $Cl^-$, $Br^-$ or $NO_3^-$,
or a salt thereof.

2. A process in accordance with claim 1, characterized in that the reaction of Grewe diamine of formula I with an orthoformic acid ester of formula II is carried out at a temperature from about 70 ° C to about 200 ° C, especially from about 90 ° C to about 150 ° C and preferably from about 100 ° C to about 120 ° C.

3. A process in accordance with claim 1 or 2, characterized in that formic acid or acetic acid is used as the acid catalyst.

4. A process in accordance with claim 3, characterized in that p-toluenesulphonic acid is used as the acid catalyst.

5. A process in accordance with any one of claims 1 to 4, characterized in that the methyl ester or the ethyl ester is used as the orthoformic acid ester of formula II.

6. A process in accordance with any one of claims 1 to 5, characterized in that the reaction of the pyrimidine derivative of formula III with a ketone of formula IV is carried out in the presence of formic acid.

**Revendications**

1. Procédé pour la préparation de thiamine ou d'un dérivé de pyrimidine, à partir de la diamine de Grewe de formule

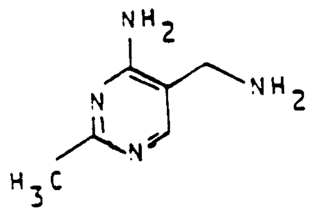

I

caractérisé en ce que l'on chauffe la diamine de Grewe avec un ester d'acide orthoformique de formule générale

$HC(OR)_3$ II

dans laquelle R représente un radical alkyle ayant de 1 à 4 atomes de carbone, en présence d'un catalyseur acide, si on le désire on fait réagir le dérivé de pyrimidine de formule

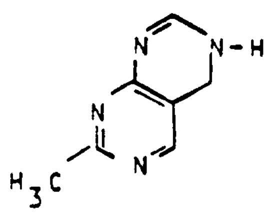

III

ainsi obtenu, avec une cétone de formule générale

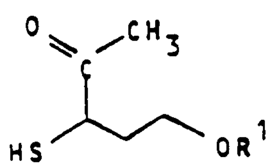

IV

dans laquelle $R^1$ représente un atome d'hydrogène ou un radical acyle ayant de 1 à 4 atomes de carbone,
en présence d'un acide carboxylique aliphatique ayant de 1 à 4 atomes de carbone, et on convertit le composé obtenu, de formule générale

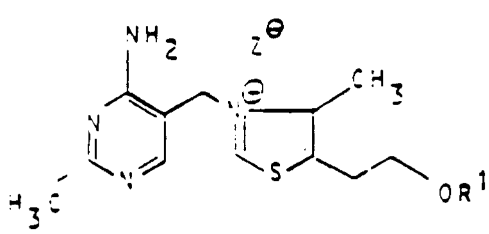

V

dans laquelle $R^1$ a la signification donnée plus haut et $Z^-$ représente l'anion de l'acide carboxylique aliphatique utilisé,
par traitement par un acide minéral, en la thiamine de formule

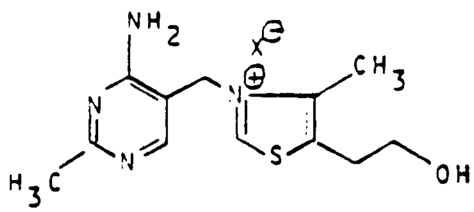

VI

dans laquelle $X^-$ représente $Cl^-$, $Br^-$ ou $NO_3^-$,
ou un sel de celle-ci.

2. Procédé selon la revendication 1, caractérisé en ce que la réaction de la diamine de Grewe de formule I avec un ester d'acide orthoformique de formule II est effectuée à une température d'environ 70 °C à environ 200 °C, en particulier d'environ 90 °C à environ 150 °C et de préférence d'environ 100 °C à environ 120 °C.

3. Procédé selon la revendication 1 où 2, caractérisé en ce que, comme catalyseur acide, on utilise l'acide formique ou l'acide acétique.

4. Procédé selon la revendication 3, caractérisé en ce que, comme catalyseur acide, on utilise l'acide p-toluène-sulfonique.

5. Procédé selon l'une des revendications 1 à 4, caractérisé en ce que, en tant qu'ester d'acide orthoformique de formule II, on utilise l'ester méthylique ou l'ester éthylique.

6. Procédé selon l'une des revendications 1 à 5, caractérisé en ce que la réaction du dérivé de pyrimidine de formule III avec une cétone de formule IV est effectuée en présence d'acide formique.